# DEMANDE DE BREVET EUROPEEN

(11) **EP 1 614 401 A1**
(43) Date de publication de la demande: **11.01.2006**
(21) Numéro de dépôt: 04291759.1
(22) Date de dépôt: 09.07.2004
(51) Int. Cl.: A61F 2/32, A61F 2/36

(54) **Prothèse de hanche et procédé de détermination de la hauteur d'une prothèse de hanche**

(71) Demandeur: De Buttet, Michel, 59170 Croix (FR)
(72) Inventeur: De Buttet, Michel, 59170 Croix (FR); Pasquier, Gilles, 59000 Lille (FR)
(74) Mandataire: Debay, Yves

(57) **Abrégé**

Prothèse de hanche comprenant un implant cotyloïdien et un implant fémoral, l'implant fémoral étant constitué d'une tige (1) prolongée par un col (2), sur lequel est impactée une tête, le col (2) se terminant par une embase (3), caractérisée en ce qu'à chaque implant fémoral sont associées au moins une cote définissant la latéralisation (6), et une cote définissant, soit la hauteur haute (7) du col (2), qui correspond à la distance comprise entre deux perpendiculaires à l'axe de la tige (1), passant respectivement par le sommet (300) de l'embase (3) d'une part, et par le centre théorique (210) de positionnement de la tête (14) d'autre part, soit la hauteur basse (8) du col (2), qui correspond à la distance comprise entre deux perpendiculaires à l'axe de la tige (1), passant respectivement par le centre théorique (210) de positionnement de la tête (14) d'une part, et un point (350) situé à l'intersection entre la ligne d'embase (3) et l'axe du col (2) d'autre part.

## Description

La présente invention concerne les prothèses de hanche. En particulier, la présente invention concerne l'adaptation de la mise en place des prothèses de hanche en fonction du patient.

Il est connu dans l'art antérieur différents types de prothèses de hanche.

Une prothèse de hanche comprend un implant fémoral et un implant cotyloïdien. On parle de prothèse de hanche totale lorsque les deux implants (fémoral et cotyloïdien) sont scellés à l'os. Ce type de prothèse est habituellement utilisé dans le cas d'affections dégénératives ou inflammatoires. On parle de prothèse de hanche intermédiaire lorsque seul l'implant fémoral est scellé. Ce type de prothèse est habituellement utilisé dans le cas de fractures du col du fémur.

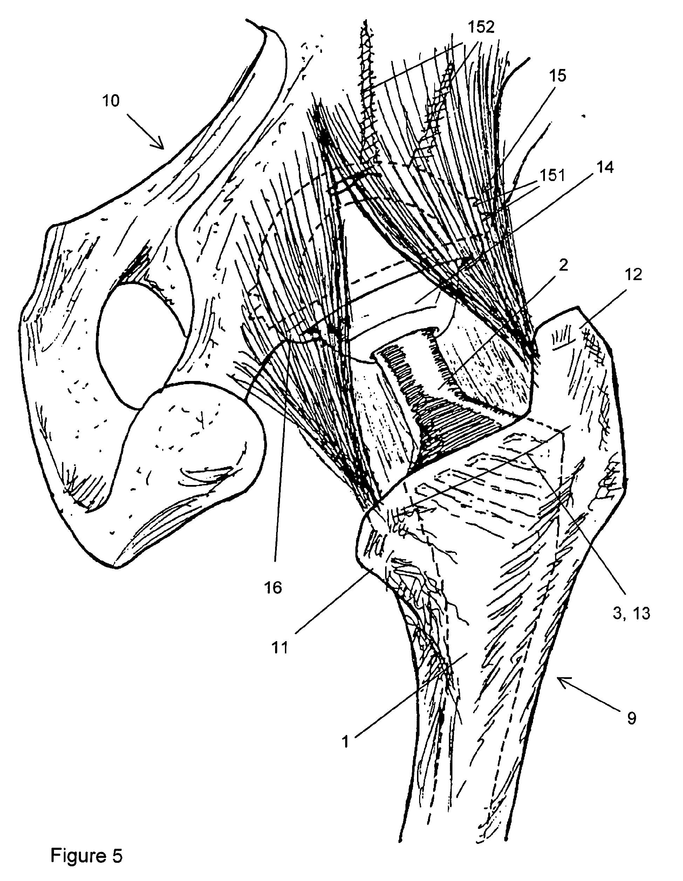

Dans le cas d'une prothèse totale, l'implant cotyloïdien est constitué d'une cupule, qui est scellée, soit par un ciment bio-compatible - la cupule est dans ce cas le plus souvent en polyéthylène - soit par un revêtement biologique, tel que l'hydroxy-apatite, un constituant de l'os, qui permet la repousse osseuse au contact de l'implant et donc l'ancrage de l'implant dans l'os. Dans le cas d'un scellement par revêtement biologique, l'insert, en métal le plus souvent, est inséré dans l'os préalablement fraisé, par impactage, ou par vissage si la surface externe de l'insert est filetée (151, figure 5), puis elle est éventuellement maintenue dans l'os à l'aide de vis (152, figure 5). Dans le cas d'une prothèse intermédiaire, l'implant cotyloïdien est constitué d'une cupule d'interface (par exemple en polyéthylène) qui est insérée dans une cupule métallique. La cupule métallique est simplement posée dans le cotyle si le cartilage du patient est en bon état, de façon à permettre une double mobilité : à la fois entre la tête de l'implant fémoral et la surface intérieure de la cupule d'interface, et entre la surface extérieure de la cupule métallique et la cavité articulaire.

L'implant fémoral est, soit monobloc, soit modulaire. Un implant fémoral modulaire est en général constitué d'une tige fémorale métallique prolongée par un col, et d'une tête qui est impactée sur le col. La tête est soit métallique, soit en céramique (de zircone ou d'alumine). La tige fémorale est, soit cimentée, soit, si elle est revêtue d'un revêtement biologique, impactée dans le fémur.

Dans toute prothèse, les surfaces de frottements doivent être compatibles et parfaitement lisses.

De plus, quelle que soit la technique de fixation de la prothèse, il est important de choisir des implants de taille adéquate, adaptés à l'anatomie du patient afin que la tenue primaire soit optimisée et que le centre de rotation de l'articulation, la longueur et la latéralisation du membre inférieur soient respectés.

Les chirurgiens font le plus souvent une planification préopératoire à partir de radiographies préopératoires et de calques de la hanche pour laquelle une prothèse est envisagée. Toutefois, depuis l'avènement de la radiographie numérique, qui remplace progressivement la radiographie argentique classique, l'utilisation systématique des calques n'est plus possible en raison d'une variabilité des coefficients d'agrandissement. Une règle centimétrique permet néanmoins de prendre des mesures, telles que la longueur du col, mais elle ne permet plus d'utiliser les calques, qui ont un coefficient d'agrandissement fixe.

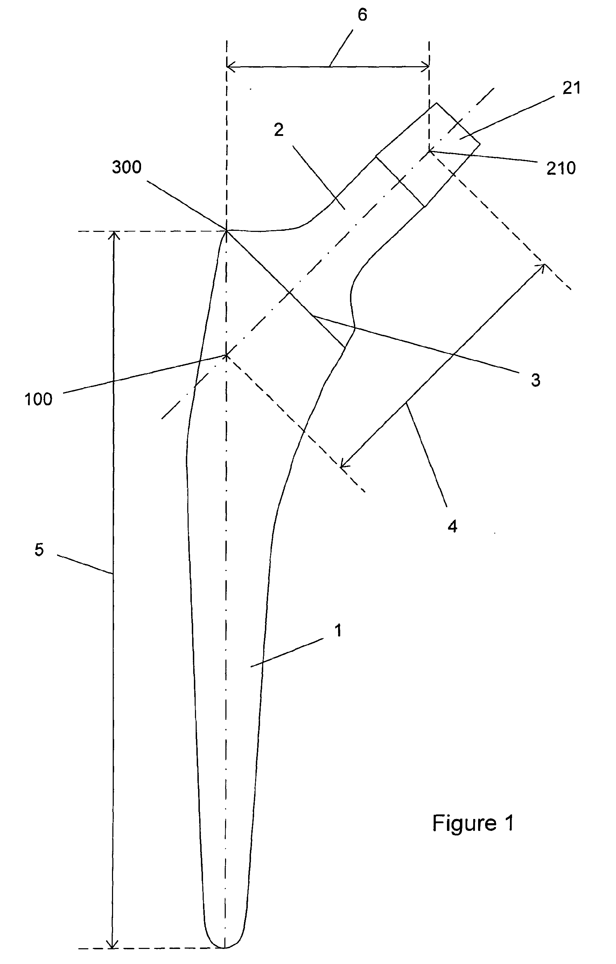

Une prothèse est habituellement caractérisée par plusieurs cotes pour permettre au chirurgien de choisir correctement une prothèse et de la mettre en place. La prothèse doit en effet permettre de rétablir la longueur et la médialisation du membre inférieur par rapport à l'autre, pour permettre une utilisation normale de la hanche sur laquelle doit être posée la prothèse. Il est donc connu dans l'art antérieur d'utiliser des cotes, comme représenté en figure 1, définissant différentes longueurs caractéristiques d'un implant fémoral. Il s'agit de la longueur (5) de la tige (1), définie comme la distance comprise entre l'extrémité inférieure de la tige (1) et le sommet (300) de l'embase, de la longueur (4) du col (2) et de la latéralisation (6) (ou offset) de l'implant.

Il existe également des cotes définissant le diamètre de la tête de l'implant fémoral à utiliser avec la tige (1). Les diamètres de tête les plus souvent utilisés sont de 22,2 mm, 28 mm et 32 mm. Ces têtes sont généralement modulaires, et permettent de modifier la longueur de l'implant fémoral. En effet, une tête dite "0" va rallonger l'implant fémoral du rayon de la tête, tandis que les têtes dites "+" et "-" vont respectivement allonger ou raccourcir la longueur totale de l'implant fémoral additionnée du rayon de la tête, d'une longueur définie par le fabricant, fonction du diamètre de la tête.

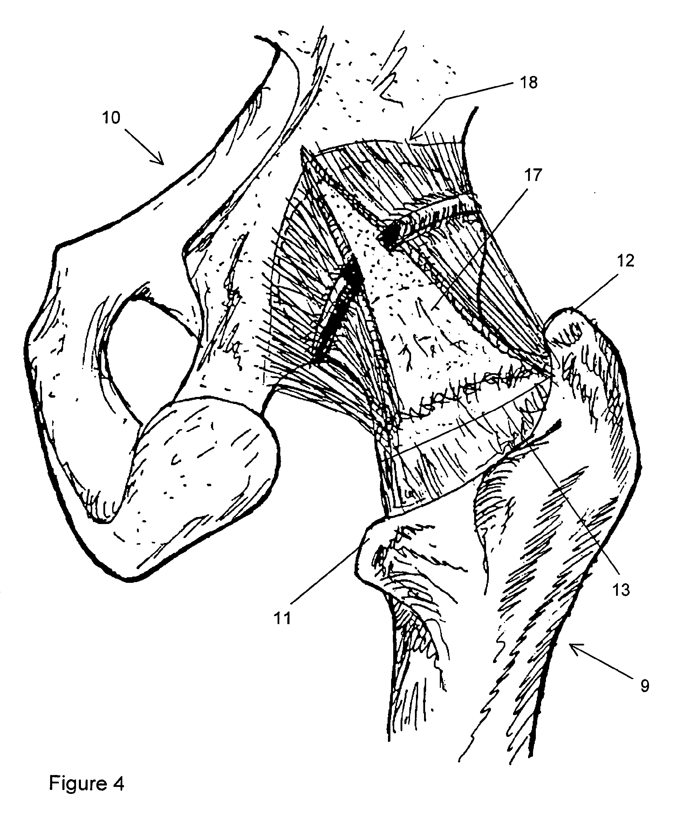

Dans l'art antérieur, la cote définissant la longueur (4) du col (2) est définie, comme représenté en figure 1, comme la distance comprise entre le centre de rotation d'une tête fémorale dite "0", positionné en haut du col (2) - et dit centre théorique (210) de positionnement - et l'intersection (100) entre l'axe de la tige (1) et l'axe du col (2). Or, avant insertion de la prothèse fémorale dans le fémur, le col du fémur est toujours ostéotomisé, sensiblement au même endroit, comme représenté en figure 4 : suivant une ligne de coupe (13) qui part environ 1 cm au dessus du petit trochanter (11) et qui atteint le grand trochanter (12) au niveau de la fossette digitale. L'implant fémoral s'insère dans le fémur de façon à ce que la ligne d'embase (3, figure 1) du col (2), à l'intersection du col (2) et de la tige (1), atteigne la ligne de coupe (13), comme représenté en figure 5. La cote définissant la longueur (4) du col (2) telle qu'enseignée par l'art antérieur ne correspond donc pas à une donnée évidente pour le positionnement en hauteur de la prothèse fémorale dans le fémur. De même, la cote définissant la latéralisation (6), qui représente la distance comprise entre le sommet (300) de l'embase (3) et une parallèle à l'axe de la tige (1) passant par le centre théorique (210) de rotation de la tête ne correspond pas à une donnée évidente pour le positionnement de la prothèse fémorale dans le fémur. Ce système de cotes est donc compliqué et oblige le chirurgien à faire des calculs ou une planification préopératoire pour mettre en place la prothèse adaptée, calculs qui ne se trouvent pas toujours validés lors de la réalisation de la mise en place des implants.

La présente invention a pour but de pallier certains inconvénients de l'art antérieur en proposant une prothèse qui soit simple d'utilisation et qui puisse être positionnée avec précision, et sans nécessairement faire des calculs ou une planification préopératoire.

Ce but est atteint par une prothèse de hanche comprenant un implant cotyloïdien et un implant fémoral, l'implant fémoral étant constitué d'une tige prolongée par un col, sur lequel est impactée une tête sphérique munie d'une ouverture cylindrique, le col se terminant par une embase coïncidant avec une ligne de coupe du col du fémur du patient lorsque l'implant fémoral est mis en place dans le fémur, caractérisée en ce que chaque implant fémoral comprend au moins un marquage de la ligne d'embase, un marquage du sommet de l'embase, et un marquage d'au moins un point de positionnement théorique du centre de la tête sur le col, dit centre théorique de positionnement de la tête, et en ce qu'à chaque implant fémoral sont associées au moins une cote définissant la latéralisation, qui correspond à la distance comprise entre le sommet de l'embase et une parallèle à l'axe de la tige passant par le centre théorique de positionnement de la tête, et une cote définissant, soit la hauteur haute du col, qui correspond à la distance comprise entre deux perpendiculaires à l'axe de la tige, passant respectivement par le sommet de l'embase d'une part, et le centre théorique de positionnement de la tête d'autre part, soit la hauteur basse du col, qui correspond à la distance comprise entre deux perpendiculaires à l'axe de la tige, passant respectivement par le centre théorique de positionnement de la tête d'une part, et un point situé à l'intersection entre la ligne d'embase et l'axe du col d'autre part.

Selon une autre particularité, chaque implant fémoral comprend un marquage du point situé à l'intersection entre la ligne d'embase et l'axe du col.

Selon une autre particularité, à chaque tête est associée une cote définissant sa hauteur en fonction, d'une part, de son diamètre, d'autre part, de sa modularité.

Selon une autre particularité, la modularité de la tête est une cote positive, négative ou nulle déplaçant le centre de la tête par rapport à son centre théorique de positionnement.

Selon une autre particularité, à chaque implant cotyloïdien est associée, si l'implant cotyloïdien est scellé et constitué d'une cupule, une cote définissant l'épaisseur de la cupule en fonction du diamètre du cotyle.

Selon une autre particularité, à chaque implant cotyloïdien est associée, si l'implant cotyloïdien est non scellé et constitué d'une première cupule insérée dans une seconde cupule :
- une cote définissant l'épaisseur de la première cupule, en fonction du diamètre du cotyle,
- une cote définissant l'épaisseur de la seconde cupule.

Selon une autre particularité, la prothèse est associée à un ou plusieurs tableaux de correspondance entre chaque taille d'implant et les cotes.

Selon une autre particularité, la hauteur de la prothèse est la somme de la partie extra-médullaire de la prothèse fémorale, à savoir la hauteur haute du col additionnée de la hauteur de la tête, et de la hauteur de la prothèse cotyloïdienne, à savoir l'épaisseur de la (des) cupule(s), la hauteur de la prothèse étant déterminée à l'aide des tableaux pour qu'elle corresponde à la hauteur séparant l'extrémité supérieure du fémur et le cotyle.

Selon une autre particularité, la hauteur de la prothèse est la somme de la partie extra-médullaire de la prothèse fémorale, à savoir la hauteur basse du col additionnée de la hauteur de la tête, et de la hauteur de la prothèse cotyloïdienne, à savoir l'épaisseur de la (des) cupule(s), la hauteur de la prothèse étant déterminée à l'aide des tableaux pour qu'elle corresponde à la hauteur séparant l'extrémité supérieure du fémur et le cotyle.

Un autre but de la présente invention est de proposer un procédé de détermination de la hauteur d'une prothèse de hanche selon l'invention en fonction du patient.

Ce but est atteint par un procédé, caractérisé en ce qu'il consiste à mesurer la hauteur séparant l'extrémité supérieure du fémur et le cotyle, ainsi que la largeur du col du fémur, sur les radiographies de la hanche du patient, à corriger ces mesures par une règle de trois en fonction de l'échelle de réduction des radiographies si elles ne sont pas à l'échelle 1, et à déterminer à l'aide des tableaux la valeur de la cote de latéralisation de la prothèse fémorale, de la cote de hauteur haute ou basse du col, correspondant respectivement à la distance comprise entre deux perpendiculaires à l'axe de la tige, passant respectivement par le sommet de l'embase d'une part, et par le centre théorique de positionnement de la tête d'autre part, ou à la distance comprise entre deux perpendiculaires à l'axe de la tige, passant respectivement par le centre théorique de positionnement de la tête d'une part, et un point situé à l'intersection entre la ligne d'embase et l'axe du col d'autre part, de la hauteur de la tête et de l'épaisseur de la (des) cupule(s), de façon à ce que l'ensemble de ces cotes correspondent aux mesures réalisées sur les radiographies du patient.

D'autres particularités et avantages de la présente invention apparaîtront plus clairement à la lecture de la description ci-après, faite en référence aux dessins annexés, dans lesquels :
- la figure 1 représente un implant fémoral standard avec le système de cotes utilisé dans l'art antérieur.
- la figure 2 représente un implant fémoral standard avec le système de cotes selon l'invention.
- la figure 3 représente un implant fémoral latéralisé avec le système de cotes selon l'invention.
- la figure 4 représente l'articulation d'une hanche avant la coupe osseuse, ainsi que la ligne de coupe du col du fémur.
- la figure 5 représente l'articulation d'une hanche après mise en place d'une prothèse de hanche.

La plupart des implants fémoraux comprennent une tige (1) prolongée par un col (2), comme représenté en particulier sur les figures 1 à 3. Le col (2) se termine par un cône morse (21), qui est un emmanchement conique de faible conicité (typiquement 5°). La tête (14) de l'implant fémoral, représentée en particulier sur la figure 5, est une sphère coupée suivant un plan, duquel part une ouverture cylindrique comprise dans la tête (14). Cette ouverture permet d'emmancher la tête (14) sur le col (2). Après positionnement de la tige (1) dans le fémur (9), la tête (14) est impactée sur le col (2) prolongeant la tige (1). La présence du cône morse (21) en extrémité du col (2) permet le blocage de la tête (14) sur ce dernier.

Comme cela a été dit plus haut, la tige (1) de l'implant fémoral est insérée dans le fémur (9) (impactée s'il s'agit d'un implant sans ciment), jusqu'à la ligne d'embase (3) de l'implant fémoral, après que le col du fémur (13) ait été ostéotomisé suivant la ligne de coupe (13) représentée en particulier sur la figure 4.

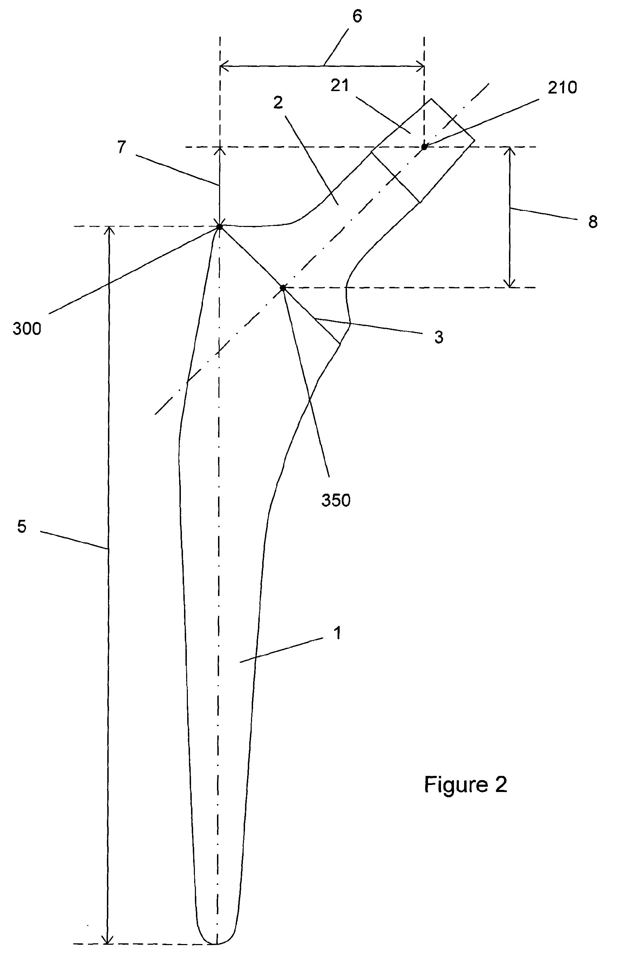

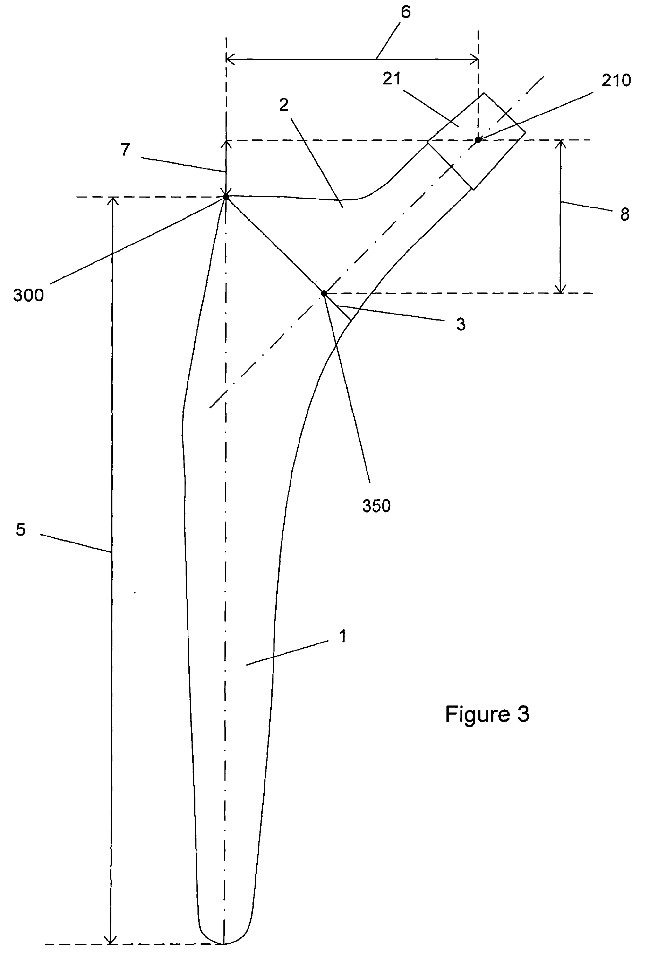

A chaque implant fémoral sont associées, selon l'invention, trois cotes (5, 6, 7) de taille, comme représenté en figures 2 et 3. Deux de ces cotes sont la longueur (5) de la tige (1) et la latéralisation (6), comme elles ont été définies dans l'art antérieur. La troisième cote selon l'invention est la hauteur dite "haute" (7) du col (2). Elle correspond à la distance comprise entre deux perpendiculaires à l'axe de la tige (1), passant respectivement par le centre théorique (210) de positionnement de la tête (14) d'une part, et par le sommet (300) de l'embase (3) d'autre part. Dans une variante de réalisation, la troisième cote selon l'invention est la hauteur dite "basse "(8) du col (2). Elle correspond à la distance comprise entre deux perpendiculaires à l'axe de la tige (1), passant respectivement par le centre théorique (210) de positionnement de la tête (14) d'une part, et le point (350) situé à l'intersection entre la ligne d'embase (3) et l'axe du col (2) d'autre part.

Selon l'invention, la ligne d'embase (3) est matérialisée sur l'implant par un trait en relief, une rainure ou un ou plusieurs points. Notons que la ligne d'embase (3) est perpendiculaire à l'axe du col (2). De même, le sommet (300) de l'embase (3), ainsi que le centre théorique (210) de positionnement de la tête (14), qui se situe sensiblement au centre du cône morse (21), et le point (350) situé à l'intersection entre la ligne d'embase (3) et l'axe du col (2) s'il s'agit de la variante décrite ci-dessus, sont matérialisés par un poinçon ou un bossage.

A chaque tête (14) est également associée, selon l'invention, une cote en millimètres définissant sa hauteur, qui est fonction, d'une part, du diamètre (typiquement de 22,2 mm, 28 mm ou 32 mm), d'autre part, de la modularité (0, +, -) de la tête (14).

Les cotes associées aux implants sont rassemblées dans un ou plusieurs tableaux comme ceux des annexes 1 à 5.

Les chiffres figurant dans les tableaux 1 à 5 ne sont que des exemples et ne sont pas limitatifs.

Les tableaux 1 à 4, respectivement en annexe 1 à 4, comprennent la cote définissant la longueur (5) de la tige (1), appelée taille de la tige. Elle est par exemple comprise entre 1 et 20. Les tableaux 1 à 4 comprennent également les cotes de hauteur haute (7) ou basse (8) du col (2) et la hauteur de la tête (14) en fonction de la taille de la tige et de la hauteur haute ou basse (7, 8) du col (2), dans le cas d'une prothèse fémorale standard (tableaux 1 et 3) et dans le cas d'une prothèse latéralisée (tableaux 2 et 4).

La cote de latéralisation (6) doit également apparaître dans un tableau selon les mêmes modalités.

La position du centre de la tête (14) peut varier de quelques millimètres par rapport au centre théorique (210) de positionnement de la tête (14) sur le cône morse (21) du col (2) de l'implant fémoral. Le système de cotes des tableaux 1 à 4 utilise donc également des cotes définissant la variation, positive, négative ou nulle, de quelques millimètres (2, 3,5 ou 7) entre le centre théorique (210) et le centre réel de la tête (14).

Ainsi, par exemple, si le chirurgien veut utiliser une tige standard de taille 7, le centre théorique (210) de la tête se situera à 12 mm au-dessus du sommet (300) de l'embase (3). S'il veut positionner une tête "0" (14) de diamètre 22,2 mm au centre théorique (210) de positionnement de la tête, le sommet de la tête se situera à 23,1 mm de l'embase (3), qui correspond à la hauteur haute (7) du col (2) additionnée de la hauteur de la tête, à savoir son rayon.

La latéralisation totale de la prothèse correspondra à la somme de cote de latéralisation (6) du col (2) et de la largeur de la tête (14), à savoir son rayon.

L'autre partie de la prothèse est l'implant cotyloïdien, qui s'insère dans le cotyle (18), dans le bassin (10). A chaque implant cotyloïdien non scellé sont associées, selon l'invention :
- une cote définissant, pour chaque diamètre de cotyle (18), l'épaisseur de la cupule métallique (15);
- une cote définissant l'épaisseur de la cupule d'interface (16) (par exemple en polyéthylène), en fonction du diamètre du cotyle (18).

A chaque implant cotyloïdien scellé, est associée, selon l'invention, une cote définissant l'épaisseur de la cupule en fonction du diamètre du cotyle.

Chaque implant est associé à au moins un tableau comme ceux représentés en annexes 1 à 5, qui rassemblent l'ensemble des informations concernant les cotes comme définies ci-dessus.

Le tableau 5, représenté en annexe 5, représente l'épaisseur de la cupule métallique (15) en fonction du diamètre du cotyle (qui varie par exemple entre 48 et 64 mm), de l'épaisseur de la cupule d'interface (16) et du diamètre de la tête (14). Ainsi, dans l'exemple du tableau 2, pour un cotyle de 54 mm de diamètre, l'épaisseur de la cupule métallique (15) est de 4,5 mm et il faut utiliser une tête de 28 mm de diamètre ; l'épaisseur de la cupule d'interface (16) est alors de 8,5 mm. Dans cet exemple, la somme des épaisseurs de la cupule métallique (15) et de la cupule d'interface (16) est alors de 13 mm.

Ainsi, grâce aux mesures prises sur les radiographies du patient ou pendant l'intervention, le chirurgien peut, par une simple règle de trois, évaluer avec précision et à l'échelle 1, les dimensions de l'articulation du patient et à partir des cotes rassemblées dans les différents tableaux, choisir la prothèse adaptée à la morphologie du patient, puis l'implanter avec précision grâce au marquage.

Dans une variante de réalisation (non représentée), le marquage de la ligne d'embase (3) est constitué de plusieurs lignes : par exemple, une pour la cote réelle et une en pointillés située 1 mm en dessous de la ligne de cote réelle, pour permettre au chirurgien de positionner, avec une précision inférieure au millimètre, la tige fémorale dans le fémur.

Au total, la hauteur d'une prothèse de hanche selon l'invention peut être définie par la somme de la partie extra-médullaire de la prothèse fémorale, à savoir la hauteur haute (7) du col (2) additionnée de la hauteur de la tête (14) (variable s'il s'agit d'une tête modulaire), et de la prothèse cotyloïdienne, à savoir l'épaisseur de la cupule (16) et, éventuellement, l'épaisseur de la cupule métallique (15) s'il s'agit d'une prothèse non scellée.

Dans une variante, la partie extra-médullaire de la prothèse fémorale à est la somme de la hauteur basse (8) du col (2), de la hauteur de la tête (14) et de l'épaisseur de la (des) cupule(s) (16, 15).

Il doit être évident pour les personnes versées dans l'art que la présente invention permet des modes de réalisation sous de nombreuses autres formes spécifiques sans l'éloigner du domaine d'application de l'invention comme revendiqué. Par conséquent, les présents modes de réalisation doivent être considérés à titre d'illustration, mais peuvent être modifiés dans le domaine défini par la portée des revendications jointes, et l'invention ne doit pas être limitée aux détails donnés ci-dessus.

**Tableau 1 ANNEXE 1 : Hauteur de la tête (14) pour un implant fémoral standard**

| Taille de la tige (1) | Hauteur haute (7) du col (2) | Hauteur de la tête (14) | | | | | | |
|---|---|---|---|---|---|---|---|---|
| | | 22,2 mm | | | 28 mm | | | |
| | | -2 | 0 | + 2 | -3,5 | 0 | + 3,5 | + 7 |
| 4 | 12 | 21,1 | 23,1 | 25,1 | 22,5 | 26 | 29,5 | 33 |
| 5 | 12 | 21,1 | 23,1 | 25,1 | 22,5 | 26 | 29,5 | 33 |
| 6 | 12 | 21,1 | 23,1 | 25,1 | 22,5 | 26 | 29,5 | 33 |
| 7 | 12 | 21,1 | 23,1 | 25,1 | 22,5 | 26 | 29,5 | 33 |
| 8 | 12 | 21,1 | 23,1 | 25,1 | 22,5 | 26 | 29,5 | 33 |
| 9 | 12 | 21,1 | 23,1 | 25,1 | 22,5 | 26 | 29,5 | 33 |
| 10 | 12 | 21,1 | 23,1 | 25,1 | 22,5 | 26 | 29,5 | 33 |
| 11 | 12 | 21,1 | 23,1 | 25,1 | 22,5 | 26 | 29,5 | 33 |

**Tableau 2 ANNEXE 2 : Hauteur de la tête (14) pour un implant fémoral latéralisé**

| Taille de la tige (1) | Hauteur haute (7) du col (2) | Hauteur de la tête (14) | | | | | | |
|---|---|---|---|---|---|---|---|---|
| | | 22,2 mm | | | 28 mm | | | |
| | | - 2 | 0 | + 2 | - 3,5 | 0 | + 3,5 | + 7 |
| 5 | 12 | 21,1 | 23,1 | 25,1 | 22,5 | 26 | 29,5 | 33 |
| 6 | 13 | 22,1 | 24,1 | 26,1 | 23,5 | 27 | 30,5 | 34 |
| 7 | 13 | 22,1 | 24,1 | 26,1 | 23,5 | 27 | 30,5 | 34 |
| 8 | 13 | 22,1 | 24,1 | 26,1 | 23,5 | 27 | 30,5 | 34 |
| 9 | 13 | 22,1 | 24,1 | 26,1 | 23,5 | 27 | 30,5 | 34 |
| 10 | 14 | 23,1 | 25,1 | 27,1 | 24,5 | 28 | 31,5 | 35 |
| 11 | 15 | 24,1 | 26,1 | 28,1 | 25,5 | 29 | 32,5 | 36 |

**Tableau 3 ANNEXE 3 : Hauteur de la tête (14) pour un implant fémoral standard**

| Taille de la tige (1) | Hauteur basse (8) du col (2) | Hauteur de la tête (14) | | | | | | |
|---|---|---|---|---|---|---|---|---|
| | | 22,2 mm | | | 28 mm | | | |
| | | -2 | 0 | + 2 | - 3,5 | 0 | + 3,5 | + 7 |
| 4 | 30 | 39,1 | 41,1 | 43,1 | 40,5 | 44 | 47,5 | 51 |
| 5 | 34 | 43,1 | 45,1 | 47,1 | 44,5 | 48 | 51,5 | 55 |
| 6 | 34 | 43,1 | 45,1 | 47,1 | 44,5 | 48 | 51,5 | 55 |
| 7 | 34 | 43,1 | 45,1 | 47,1 | 44,5 | 48 | 51,5 | 55 |
| 8 | 34 | 43,1 | 45,1 | 47,1 | 44,5 | 48 | 51,5 | 55 |
| 9 | 34 | 43,1 | 45,1 | 47,1 | 44, 5 | 48 | 51,5 | 55 |
| 10 | 35 | 44,1 | 46,1 | 48,1 | 45,5 | 49 | 52,5 | 56 |
| 11 | 35 | 44,1 | 46,1 | 48,1 | 45,5 | 49 | 52,5 | 56 |

**Tableau 4 ANNEXE 4 : Hauteur de la tête (14) pour un implant fémoral latéralisé**

| Taille de la tige (1) | Hauteur basse (8) du col (2) | Hauteur de la tête (14) | | | | | | |
|---|---|---|---|---|---|---|---|---|
| | | 22,2 mm | | | 28 mm | | | |
| | | - 2 | 0 | +2 | -3,5 | 0 | +3,5 | +7 |
| 5 | 39 | 48,1 | 50,1 | 52,1 | 49,5 | 53 | 56,5 | 60 |
| 6 | 39 | 48,1 | 50,1 | 52,1 | 49,5 | 53 | 56,5 | 60 |
| 7 | 39 | 48,1 | 50,1 | 52,1 | 49,5 | 53 | 56,5 | 60 |
| 8 | 39 | 48,1 | 50,1 | 52,1 | 49,5 | 53 | 56,5 | 60 |
| 9 | 40 | 49,1 | 51,1 | 53,1 | 50,5 | 54 | 57,5 | 61 |
| 10 | 40 | 49,1 | 51,1 | 53,1 | 50,5 | 54 | 57,5 | 61 |
| 11 | 41 | 50,1 | 52,1 | 54,1 | 51,5 | 55 | 58,5 | 62 |

**Tableau 5 ANNEXE 5**

| Diamètre du cotyle | Epaisseur de la cupule métallique | Epaisseur de la cupule d'interface (en mm) pour | | | |
|---|---|---|---|---|---|
| | | tête de 22,2 mm | | tête de 28 mm | |
| | | Polyéthylène | total | Polyéthylène | total |
| 48 | 4,5 | 8,4 | 12,9 | 5,5 | 10,0 |
| 50 | 4,5 | 9,4 | 13,9 | 6,5 | 11,0 |
| 52 | 4,5 | - | - | 7,5 | 12,0 |
| 54 | 4,5 | - | - | 8,5 | 13,0 |
| 56 | 4,5 | - | - | 9,5 | 14,0 |
| 58 | 4,5 | - | - | 10,5 | 15,0 |
| 60 | 4,5 | - | - | 11,5 | 16,0 |
| 62 | 4,5 | - | - | 12,5 | 17,0 |
| 64 | 4,5 | - | - | 13,5 | 18,0 |
| 64 | 4,5 | - | - | 14,5 | 19,0 |

## Revendications

1. Prothèse de hanche comprenant un implant cotyloïdien et un implant fémoral, l'implant fémoral étant constitué d'une tige (1) prolongée par un col (2), sur lequel est impactée une tête (14) sphérique munie d'une ouverture cylindrique, le col (2) se terminant par une embase (3) coïncidant avec une ligne de coupe (13) du col du fémur (17) du patient lorsque l'implant fémoral est mis en place dans le fémur (9), **caractérisée en ce que** chaque implant fémoral comprend au moins un marquage de la ligne d'embase (3), un marquage du sommet (300) de l'embase (3), et un marquage d'au moins un point de positionnement théorique du centre de la tête (14) sur le col (2), dit centre théorique (210) de positionnement de la tête (14), et **en ce qu'**à chaque implant fémoral sont associées au moins une cote définissant la latéralisation (6), qui correspond à la distance comprise entre le sommet (300) de l'embase (3) et une parallèle à l'axe de la tige (1) passant par le centre théorique (210) de positionnement de la tête (14), et une cote définissant, soit la hauteur haute (7) du col (2), qui correspond à la distance comprise entre deux perpendiculaires à l'axe de la tige (1), passant respectivement par le sommet (300) de l'embase (3) d'une part, et par le centre théorique (210) de positionnement de la tête (14) d'autre part, soit la hauteur basse (8) du col (2), qui correspond à la distance comprise entre deux perpendiculaires à l'axe de la tige (1), passant respectivement par le centre théorique (210) de positionnement de la tête (14) d'une part, et un point (350) situé à l'intersection entre la ligne d'embase (3) et l'axe du col (2) d'autre part.

2. Prothèse selon la revendication 1, **caractérisée en ce que** chaque implant fémoral comprend un marquage du point (350) situé à l'intersection entre la ligne d'embase (3) et l'axe du col (2).

3. Prothèse selon la revendication 1 ou 2, **caractérisée en ce qu'**à chaque tête (14) est associée une cote définissant sa hauteur en fonction, d'une part, de son diamètre, d'autre part, de sa modularité.

4. Prothèse selon la revendication 3, **caractérisée en ce que** la modularité de la tête (14) est une cote positive, négative ou nulle déplaçant le centre de la tête (14) par rapport à son centre théorique (210) de positionnement.

5. Prothèse selon une des revendications 1 à 4, **caractérisée en ce qu'**à chaque implant cotyloïdien est associée, si l'implant cotyloïdien est scellé et constitué d'une cupule, une cote définissant l'épaisseur de la cupule en fonction du diamètre du cotyle.

6. Prothèse selon une des revendications 1 à 4, **caractérisée en ce qu'**à chaque implant cotyloïdien est associée, si l'implant cotyloïdien est non scellé et constitué d'une première cupule (16) insérée dans une seconde cupule (15) :
- une cote définissant l'épaisseur de la première cupule (16), en fonction du diamètre du cotyle (18),
- une cote définissant l'épaisseur de la seconde cupule (15).

7. Prothèse selon une des revendications 1 à 6, **caractérisée en ce qu'**elle est associée à un ou plusieurs tableaux de correspondance entre chaque taille d'implant et les cotes.

8. Prothèse selon la revendication 7, **caractérisée en ce que** la hauteur de la prothèse est la somme de la partie extra-médullaire de la prothèse fémorale, à savoir la hauteur haute (7) du col (2) additionnée de la hauteur de la tête (14), et de la hauteur de la prothèse cotyloïdienne, à savoir l'épaisseur de la (des) cupule(s) (16, 15), la hauteur de la prothèse étant déterminée à l'aide des tableaux pour qu'elle corresponde à la hauteur séparant l'extrémité supérieure du fémur (9) et le cotyle (18).

9. Prothèse selon la revendication 7, **caractérisée en ce que** la hauteur de la prothèse est la somme de la partie extra-médullaire de la prothèse fémorale, à savoir la hauteur basse (8) du col (2) additionnée de la hauteur de la tête (14), et de la hauteur de la prothèse cotyloïdienne, à savoir l'épaisseur de la (des) cupule(s) (16, 15), la hauteur de la prothèse étant déterminée à l'aide des tableaux pour qu'elle corresponde à la hauteur séparant l'extrémité supérieure du fémur (9) et le cotyle (18).

10. Procédé de détermination de la hauteur d'une prothèse de hanche selon une des revendications 1 à 9 en fonction du patient, **caractérisé en ce qu'**il consiste à mesurer la hauteur séparant l'extrémité supérieure du fémur (9) et le cotyle (18), ainsi que la largeur du col du fémur (17), sur les radiographies de la hanche du patient, à corriger ces mesures par une règle de trois en fonction de l'échelle de réduction des radiographies si elles ne sont pas à l'échelle 1, et à déterminer à l'aide des tableaux la valeur de la cote de latéralisation (6) de la prothèse fémorale, de la cote de hauteur haute ou basse (7 ou 8) du col (2), correspondant respectivement à la distance comprise entre deux perpendiculaires à l'axe de la tige (1), passant respectivement par le sommet (300) de l'embase (3) d'une part, et par le centre théorique (210) de positionnement de la tête (14) d'autre part, ou à la distance comprise entre deux perpendiculaires à l'axe de la tige (1), passant respectivement par le centre théorique (210) de positionnement de la tête (14) d'une part, et un point (350) situé à l'intersection entre la ligne d'embase (3) et l'axe du col (2) d'autre part, de la hauteur de la tête (14) et de l'épaisseur de la (des) cupule(s) (16, 15), de façon à ce que l'ensemble de ces cotes correspondent aux mesures réalisées sur les radiographies du patient.
